# EUROPÄISCHE PATENTSCHRIFT

(11) **EP 2 662 146 B1**
(45) Veröffentlichungstag und Bekanntmachung des Hinweises auf die Patenterteilung: **26.02.2020**
(21) Anmeldenummer: 13165279.4
(22) Anmeldetag: 25.04.2013
(51) Int. Cl.: B05B 7/24, B05B 15/00, A61M 1/00, A61M 11/02, A61M 3/02, B05B 15/50, B05B 14/30, B05B 7/12, B05B 12/00, A61B 17/00

(54) **Lavage-System mit Düse**
Lavage system with nozzle
Système de lavage avec buse

(30) Priorität: 07.05.2012 DE 102012013464
(43) Veröffentlichungstag der Anmeldung: 13.11.2013
(73) Patentinhaber: Heraeus Medical GmbH, 61273 Wehrheim (DE)
(72) Erfinder: Vogt, Sebastian, 99092 Erfurt (DE)
(74) Vertreter: Sterzel, Roland

(56) Entgegenhaltungen:
- EP-A1- 0 478 448
- EP-A1- 1 769 886
- WO-A1-98/01181
- WO-A1-2010/081244
- WO-A2-00/27339
- DE-A1- 2 808 499
- FR-A1- 2 296 472
- JP-U- H0 652 839
- US-A- 4 692 140
- US-A- 5 520 667

## Beschreibung

Die Erfindung betrifft eine medizinische Sprühvorrichtung, insbesondere LavageSystem umfassend eine Druckgaszuleitung zum Erzeugen eines Gasstroms, eine Flüssigkeitszuleitung zum Zuführen einer Flüssigkeit, ein Austragsrohr mit einem offenen Rohrende zum Aussprühen von feinen Tröpfchen oder eines Sprühnebels eines Flüssigkeits-Gas-Gemischs und ein bedienbares Ventilelement, das in der Druckgaszuleitung angeordnet ist. Die Erfindung betrifft ferner ein Sprühvorrichtungs-Kit umfassend eine solche medizinische Sprühvorrichtung.

Medizinische Sprühvorrichtungen werden zum Erzeugen eines Sprühstrahls oder Sprühkegels aus einem Gas-Flüssigkeits-Gemisch verwendet, der zum Reinigen einer Wunde, einer Kavität, eines Implantats oder eines Körperteils dient.

Lavage-Systeme werden als medizinische Sprühvorrichtungen in der Chirurgie in breitem Umfang eingesetzt, um Gewebeareale zu reinigen. Insbesondere bei der Implantation von Gelenkendoprothesen und bei septischen Revisionen sind Lavage-Systeme von wesentlicher Bedeutung (R. M. Sherman et al.: "The role of lavage in preventing hemodynamic and blood-gas changes during demented arthroplasty" J. Bone Joint. Surg. 1983; 65-A: Seiten 500-506; S. J. Breusch et al.: Zementierte Hüftendoprothetik: Verminderung des Fettembolierisikos in der zementierten Hüftendoprothetik mittels gepulster Druckspülung, Orthopädie 2000; 29: Seiten 578-586; S. J. Breusch et al.: Lavage technique in THA: Jet-lavage Produces Better Cement Penetration Than Syringe- Lavage in the Proximal Femur; J. Arthroplasty. 200; 15(7): Seiten 921-927; R. J. Byrick et al.: High-volume, high pressure pulsatile lavage during cemented arthroplasty, J. Bo- ne Joint Surg. 1989; 81-A: Seiten 1331-1336 und J. Christie et al.: Medullary lavage reduces embolic phenomena and cardiopulmonary changes during cemented hemi- arthorplasty, J. Bone Joint Surg. 1995; 77-B: Seiten 456-459) Die Reinigung der Gewebeareale erfolgt bei der Lavage durch Sprühstöße mit geeigneten Spülflüssigkeiten, wie isotonische Kochsalzlösungen. Üblich sind dabei bis zu mehreren tausend Sprühstößen pro Minute.

Gepulste Lavage-Systeme sind seit langem bekannt. Beispielhaft seien dafür die Offenlegungsschriften US 4,583,531 A, US 4,278,078 A und US 5,542,918 A genannt. Die gegenwärtig im Markt befindlichen Lavagesysteme werden durch Elektromotoren (zum Beispiel InterPulse® Jet lavage der Firma Stryker GmbH & Co. KG) oder durch Druckluft (zum Beispiel PALAVAGE® der Heraeus Medical GmbH) angetrieben.

Die US 2003 036 723 A1 offenbart ein Lavage-System mit einer Pumpe, die mit einem Elektromotor über ein Getriebe angetrieben wird. Die Pumpe erzeugt den Flüssigkeits-strahl. Die Energie für den Motor wird über ein Stromkabel zugeführt. Die WO 00/27339 A2 und die WO 98/01181 A1 offenbaren Wundspülsysteme, die an einer externen Luftdruckquelle angeschlossen werden, um eine medizinische Spülflüssigkeit aus einem Behälter auszutreiben und mit der Spülflüssigkeit einen Sprühstrahl zu erzeugen. Die DE 28 08 499 A1 offenbart einen Inhalator zum Erzeugen eines Flüssigkeitsnebels.

Die DE 10 2009 021 421 A1 schlägt eine Jet-Lavage mit einem Hubmagneten vor, der die Membranpumpe antreibt.

Bei den druckluftgetriebenen Systemen wird üblicherweise ein Druckluftmotor zum Antrieb eingesetzt. Bei den meisten Systemen handelt es sich dabei um einen Lamellen-Druckluftmotor. Der Druckluftmotor erzeugt eine Drehbewegung, die dann in eine oszillierende, lineare Bewegung umgesetzt wird. Die oszillierende, lineare Bewegung wird genutzt, um jeweils kleinen Volumina des Spülmediums einen Impuls zu verleihen. Dabei wird üblicherweise mindestens eine Membran zwischen dem Antrieb und dem Zulauf der Spülflüssigkeit angeordnet, um die Impulse auf die Spülflüssigkeit übertragen zu können. Es entstehen dadurch Sprühstöße. Bei hohen Pulszahlen von 2000 bis 3000 Impulsen pro Minute werden dabei Volumina im Bereich von mehreren hundert Millilitern Spülflüssigkeit versprüht. Das bedeutet, dass der Druckluftmotor sehr präzise gefertigt sein muss, um entsprechend hohe Drehzahlen zu tolerieren. Weiterhin muss eine entsprechend stabile Lagerung vorhanden sein. Aus diesen Gründen ist der Druckluftmotor bei üblichen Druckluft-getriebenen Lavage-Systemen das kostenintensivste Bauteil. Es wird deshalb im Allgemeinen der Druckluftmotor in einem Handgriff aus Metall oder anderen langzeitstabilen Materialien angeordnet, so dass dieses Bauteil mehrfach nach entsprechender Aufbereitung und Sterilisation genutzt werden kann.

Bewährt haben sich auch batteriebetriebene Lavage-Systeme. Es muss jedoch immer ein großer Batterieblock mitgeführt werden, der jedoch naturgemäß eine begrenzte Ladungskapazität hat. Druckluftgetriebene Lavage-Systeme haben dagegen den Vorteil, dass Druckluft in vielen Operationssälen unlimitiert zur Verfügung steht und damit beliebig lange Spülflüssigkeit versprüht werden kann, ohne dass die Energiezufuhr begrenzt ist. Problematisch ist dabei jedoch, dass immer ein Druckluftschlauch das Lavage-System mit der stationären Druckluftleitung verbinden muss.

Als Flüssigkeit für Lavage-Systeme können steriles Wasser und wässrige Lösungen, die auch pharmazeutisch wirksame Substanzen enthalten können, verwendet werden. Das Spülwasser mit den Rückständen kann durch das Lavage-System über einen zweiten Kanal abgesaugt werden.

Aus der DE 10 2010 046 057 B3 ist ein gattungsgemäßes Lavage-System bekannt, bei dem ein linear oszillierender Stößel durch einen Druckgasmotor angetrieben wird, dem über ein bedienbares Ventil Druckluft zuführbar ist. Der Stößel schlägt periodisch auf eine Membran des Lavage-Systems und bildet dadurch eine periodisch arbeitende MembranPumpe zum Erzeugen des pulsierenden Flüssigkeitsstrahls.

Nachteilig ist hieran der komplexe Aufbau des Lavage-Systems. Aufgrund der hygienischen Anforderungen müssen zumindest Teile des Lavage-Systems sterilisierbar sein oder das Lavage-System muss ein möglichst kostengünstiges Wegwerfprodukt sein.

Aufgabe der Erfindung ist es, diese Nachteile zu überwinden. Insbesondere soll ein kostengünstigeres System bereitgestellt werden, das einfach eingesetzt und ressourcenschonend aufgebaut werden kann.

Der Erfindung liegt ferner die Aufgabe zu Grunde, eine einfache medizinische Sprühvorrichtung zu entwickeln, die aus möglichst wenigen Teilen besteht und die durch komprimiertes Gas angetrieben wird. Die Antriebsvorrichtung soll weitgehend aus kostengünstigen Materialien bestehen, die durch einfache Verfahren gefertigt werden können. Damit soll es möglich sein, eine medizinische Sprühvorrichtung für den einmaligen Gebrauch mit geringen Herstellungs- und Montagekosten bereitzustellen. Es sollen kostenintensive, rotierende oder bewegliche Teile, die eine präzise, stabile Lagerung erfordern, möglichst vermieden werden.

Diese Aufgaben werden gelöst durch eine medizinische Sprühvorrichtung, insbesondere ein Lavage-System, umfassend eine Druckgaszuleitung zum Führen eines Gasstroms, eine Flüssigkeitszuleitung zum Zuführen einer Flüssigkeit, ein Austragsrohr mit einem offenen Rohrende zum Aussprühen von feinen Tröpfchen oder eines Sprühnebels eines Flüssigkeits-Gas-Gemischs und ein bedienbares Ventilelement, das in der Druckgaszuleitung angeordnet ist, wobei in dem Austragsrohr eine Verengung der Querschnittsfläche vorgesehen ist und eine Einmündung im Bereich der Verengung angeordnet ist, durch die die Flüssigkeit in den Gasstrom aus der Druckgaszuleitung mündet, wobei die Verengung in dem Austragsrohr eine Düse bildet, und wobei an dem Ende der Druckgaszuleitung, durch die das strömende Gas die Druckgaszuleitung Richtung der Verengung verlässt, eine zweite Düse angeordnet ist.

Die Verengung der Querschnittsfläche reduziert dabei den inneren Querschnitt des Ausströmrohrs. Es ist also der Leitungsquerschnitt der Leitung, die durch das Ausströmrohr gebildet wird, bereichsweise reduziert.

Dabei kann vorgesehen sein, dass im Bereich des offenen Rohrendes des Austragsrohrs eine Absaugeinrichtung vorgesehen ist, die an eine Absaugleitung angeschlossen ist, wobei bevorzugt die Absaugeinrichtung ein Absaugrohr umfasst, in das die Absaugleitung mündet, und besonders bevorzugt das Absaugrohr das Austragsrohr umgibt.

Mit der Absaugeinrichtung können Rückstände, die freigesprüht wurden, abgesaugt werden.

Ferner kann vorgesehen sein, dass an dem äußersten Rohrende, insbesondere an dem Austragsrohr oder an der Absaugeinrichtung ein Trichter angeordnet ist, der zur Begrenzung und/oder Ausrichtung des erzeugten Sprühkegels geeignet ist.

Wenn die Sprühvorrichtung gleichzeitig über eine Absaugeinrichtung verfügt, wird auch die Saugwirkung durch den Trichter gerichtet.

Gemäß einer besonders bevorzugten Ausgestaltung der Erfindung kann vorgesehen sein, dass das Ventilelement ein Drehventil ist, insbesondere ein rotationssymmetrisches Drehventil, vorzugsweise ein konisches oder zylindrisches Drehventil mit einem

Durchgang, wobei das Drehventil um die Drehachse drehbar in einer Ausnehmung in einem Ausströmkörper, der einen Teil der Druckgaszuleitung definiert, gelagert ist, wobei bevorzugt das Drehventil durch einen Abzug bedienbar ist, besonders bevorzugt das Drehventil einteilig mit dem Abzug ausgebildet ist.

Derart aufgebaute Drehventile sind kostengünstig herzustellen und können leicht in entsprechende Ausnehmungen der Sprühvorrichtungen eingesetzt werden. Grundsätzlich sind auch andere Ventilelemente vorstellbar, die beispielsweise auch elektrisch gesteuert werden können. Solche Ventilelemente sind jedoch aufwendiger in der Herstellung und daher kostenintensiver. Zudem muss dann gegebenenfalls eine Spannungsquelle vorgesehen sein.

Als Ventilelement ist also ein Drehventil besonders bevorzugt. Drehventile bestehen im einfachsten Fall nur aus einem Zylinder oder Konus, der mindestens eine durchgängige Bohrung enthält, wobei der Zylinder oder Konus in einem einfachen zylinderförmigen oder konusförmigen Ventilsitz angeordnet ist. Drehventile lassen sich daher kostengünstig relativ einfach durch Spritzgießen von Kunststoffen herstellen.

Eine weitere Ausgestaltung einer erfindungsgemäßen Sprühvorrichtung kann vorsehen, dass das Ventilelement durch ein Federelement im geschlossenen Zustand gehalten wird, wobei vorzugsweise das Federelement auf einer Seite am Abzug befestigt ist.

Ein solcher Aufbau ist bedienungsfreundlicher, als wenn das Ventilelement aktiv geschlossen werden muss.

Besonders bevorzugt kann vorgesehen sein, dass die Sprühvorrichtung im Wesentlichen aus Kunststoff besteht, bevorzugt thermoplastischem Kunststoff, besonders bevorzugt gammasterilisierbarem thermoplastischem Kunststoff, wobei insbesondere das Austragsrohr, das Ventilelement, die Druckgaszuleitung, der Trichter, das Absaugrohr, der Ausströmkörper und/oder die Verengung aus Kunststoff bestehen.

Kunststoffe sind kostengünstig in der Fertigung. Durch die Verwendung von thermoplastischen Kunststoffen kann die Sprühvorrichtung durch Spritzgießen kostengünstig in großen Stückzahlen gefertigt werden. Dadurch ist es möglich die Sprühvorrichtung so kostengünstig herzustellen, dass diese als Einmalartikel eingesetzt werden kann.

Gammasterilisierbare Kunststoffe sind für medizinische Anwendungen besonders geeignet, um die hohen klinischen Anforderungen an Medizinprodukte für den Operationsbereich zu erfüllen.

Gemäß einer besonders kostengünstig aufzubauenden Variante der Erfindung kann vorgesehen sein, dass die Druckgaszuleitung zumindest bereichsweise als Ausströmrohr ausgebildet ist, das zumindest bereichsweise, insbesondere vollständig von dem Austragsrohr umgeben ist, wobei vorzugsweise die Druckgaszuleitung durch eine Zuleitungsdüse in das Austragsrohr mündet.

Eine Weiterbildung der Erfindung kann vorsehen, dass die Einmündung bezogen auf die Strömungsrichtung vor der Düse angeordnet ist.

Insbesondere dabei kann vorgesehen sein, dass die Verengung durch einen runden Ring mit dreieckiger Querschnittsfläche realisiert wird, wobei eine der Ecken der dreieckigen Querschnittsfläche in Richtung der Ringmitte orientiert ist.

Alternativ dazu kann vorgesehen sein, dass die Einmündung in der Verengung ausgebildet ist, wobei bevorzugt die Düse eine Venturi-Düse ist.

Hierdurch kann ein besonders genau gerichteter Strahl beziehungsweise Kegel erzeugt werden.

Auch kann vorgesehen sein, dass die Sprühvorrichtung einen Druckgasbehälter umfasst, der vorzugsweise mit flüssigem Kohlendioxid gefüllt ist, wobei der Druckgasbehälter bevorzugt in einem Griffstück angeordnet ist.

Diese Ausführungsform ist unabhängig davon, ob im Einsatzbereich eine Druckgaszuleitung oder ein Kompressor vorhanden ist oder nicht. Insbesondere für den mobilen Einsatz sind solche Sprühvorrichtungen besonders gut geeignet.

Eine besonders bevorzugte Weiterbildung der Erfindung kann vorsehen, dass die Sprühvorrichtung einen einstellbaren Druckminderer aufweist, mit dem der Gasdruck in die Druckgaszuleitung einstellbar ist.

Der Druck des Strahls beziehungsweise des Sprühkegels kann dadurch auf das zu behandelnde Gewebe oder Material abgestimmt werden. Eine solche Sprühvorrichtung erlaubt daher eine breitere Anwendung.

Auch ist vorgesehen, dass an dem Ende der Druckgaszuleitung, durch die das strömende Gas die Druckgaszuleitung in Richtung der Verengung verlässt, die zweite Düse angeordnet ist, bevorzugt durch das Ausströmrohr ausgeformt ist.

Durch diese zweite Düse wird eine Gasströmung erzeugt, die besonders gut zum Erzeugen des Gas-Flüssigkeits-Sprühnebels geeignet ist und die ein chaotisches, pulsartiges Ausstoßen von Flüssigkeitsschüben fördert.

Die Aufgaben der Erfindung werden auch gelöst durch ein Sprühvorrichtungs-Kit umfassend eine solche medizinische Sprühvorrichtung, eine Gaszuleitung, insbesondere flexible Gaszuleitung, einen Druckregler und einen Druckgasbehälter.

Der Erfindung liegt die überraschende Erkenntnis zugrunde, dass es durch die Verengung in dem Austragsrohr und den beim Durchströmen der Verengung auftretenden Kräften gelingt, einen Gas-Flüssigkeits-Sprühstrahl beziehungsweise einen Gas- Flüssigkeits-Sprühkegel zu erzeugen, bei dem Flüssigkeitstropfen von der Gasströmung mitgerissen werden. Der erzeugte Sprühstrahl ist zur medizinischen Behandlung geeignet. Durch die stochastische und chaotische Mitnahme von Flüssigkeitstropfen entstehen nicht periodische Pulse, die überraschend zu einer guten Reinigungswirkung führen, wie Experimente der Erfinder gezeigt haben. Gleichzeitig ist der Sprühstrahl aber konstanter und dadurch in kürzerer Zeit eine vergleichbare Reinigungswirkung erzielbar im Vergleich zu bekannten Lavage-Systemen beziehungsweise medizinischen Sprühvorrichtungen.

Die medizinische Sprühvorrichtung basiert darauf, dass zuerst komprimiertes Gas durch die Druckgaszuleitung zu dem Ventilelement geleitet wird. Durch Öffnen des Ventilelements strömt das komprimierte Gas in das Ausströmrohr und danach durch ein als zweite Düse ausgebildetes offenes Ende des Ausströmrohrs in das Austragsrohr, wobei es die Verengung passiert. Durch die Verengung erhöht sich die Strömungsgeschwindigkeit des Gases beziehungsweise des Gas-Flüssigkeits-Gemischs und es entsteht ein Unterdruck senkrecht zur Strömungsrichtung. Durch diesen Unterdruck wird Flüssigkeit aus dem Flüssigkeitszulauf angesaugt. Die angesaugte Flüssigkeit bewegt sich in Richtung der durch die Verengung gebildeten Düse und vermischt sich mit dem strömenden Gasstrom. Es entsteht ein Flüssigkeits-Gas-Sprühstrahl. Dieser Sprühstrahl enthält fein verteilte Flüssigkeitströpfchen und verlässt das Austragsrohr an seinem offenen Rohrende.

Eine erfindungsgemäße medizinische Sprühvorrichtung kann also beispielsweise aufgebaut sein aus:
a) einer Druckgaszuleitung,
b) einem Ventilelement, das mit der Druckgaszuleitung verbunden ist,
c) einem mit dem Ventilelement verbundenen, hohlem Ausströmrohr, das an seinem dem Ventilelement abwandten Ende als Düse ausgebildet ist,
d) einem Austragsrohr mit einem offenen Rohrende und einem geschlossenen Rohrende, welches das Ausströmrohr umschließt,
e) mindestens einer Verengung im Austragsrohr, wobei die Verengung in Richtung des offenen Rohrendes gegenüber der Düse des Ausströmrohrs angeordnet ist, und
f) einem Flüssigkeitszulauf der mit dem Austragsrohr flüssigkeitsdurchlässig verbunden ist, wobei der Flüssigkeitszulauf bezogen auf das offene Ende des Rohres hinter der Düse des Ausströmrohrs angeordnet ist.

Die erfindungsgemäße Antriebsvorrichtung kann auf Grund ihrer Einfachheit vorteilhaft als Einmalartikel gefertigt werden.

Zur Steuerung der Geometrie des Sprühstrahls kann gegebenenfalls auf das offene Ende des Austragsrohrs eine dritte Düse aufgesetzt werden. Diese dritte Düse kann eine oder mehrere Öffnungen enthalten, die entsprechend der gewünschten Geometrie des Sprühstrahls angeordnet sind.

Erfindungsgemäß kann auch vorgesehen sein, dass die Sprühvorrichtung in einem Gehäuse angeordnet ist, wobei das Gehäuse zumindest bereichsweise als Handgriff ausgebildet ist, besonders bevorzugt als pistolenartiger Handgriff ausgebildet ist.

Das Ventilelement ist vorteilhaft mit einem Abzug verbunden, der durch eine Feder in den geschlossenen Zustand gedrückt wird. Die Feder und Teile des Abzugs sind dabei innerhalb des Gehäuses gelagert.

Ein erfindungsgemäßes Sprühvorrichtungs-Kit mit einer erfindungsgemäßen Sprühvorrichtung kann ferner eine flexible Gaszuleitung, einen Druckregler und einen Druckgasbehälter umfassen.

Als Druckgasbehälter beziehungsweise Druckgaspatronen kommen alle mit ungiftigen Gasen gefüllte Patronen, insbesondere Metallpatronen in Frage. Besonders bevorzugt sind jedoch mit flüssigem Kohlendioxid gefüllte Patronen. Kohlendioxid ist ungiftig, nicht brennbar, nicht explosiv und kostengünstig. Mit flüssigem Kohlendioxid lässt sich ein großes Gasvolumen in kleinen Patronen speichern.

Der besondere Vorteil des Sprühvorrichtungs-Kits besteht darin, dass keine stationären Druckluftzuleitungen notwendig sind und trotzdem ein kraftvoller Sprühstrahl möglich ist. Durch die Verwendung von Druckgas gibt es keine Brand- oder Explosionsgefahr, wie sie bei elektrischen Antriebsvorrichtungen vorkommen kann. Batterie- oder Akkumulatorenblöcke sind ebenfalls nicht erforderlich.

Die verschiedenen Ausführungsformen der Erfindung stellen einfache Realisierungsvarianten des Erfindungsgedankens dar. Der Aufbau der Sprühvorrichtung bleibt in allen Fällen denkbar einfach.

Im Folgenden werden Ausführungsbeispiele der Erfindung anhand von zwei schematisch dargestellten Figuren erläutert, ohne jedoch dabei die Erfindung zu beschränken. Dabei zeigt:
- Figur 1:: eine schematische Querschnittansicht einer erfindungsgemäßen Sprühvorrichtung;
- Figur 2:: eine schematische Querschnittansicht einer alternativen erfindungsgemäßen Sprühvorrichtung mit Absaugeinrichtung; und
- Figur 3:: eine schematische Querschnittansicht einer dritten Sprühvorrichtung mit einer Venturi-Düse.

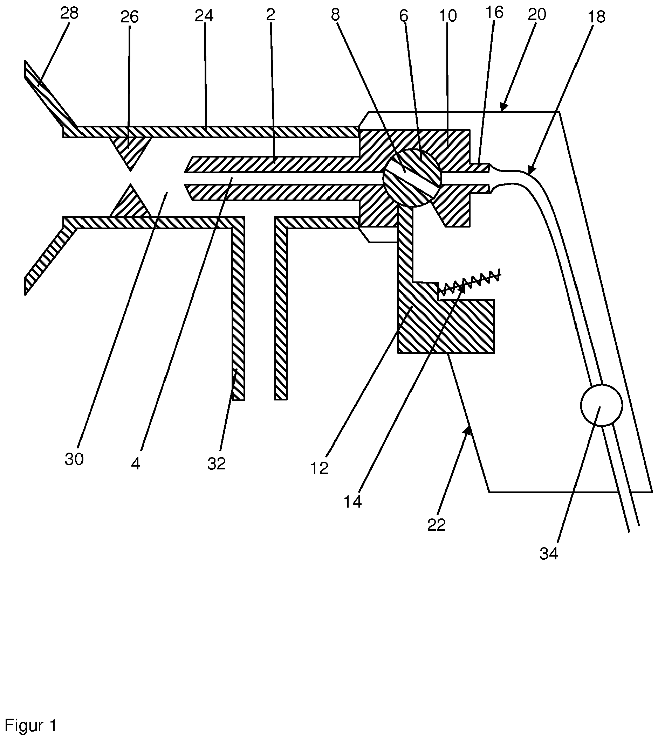

Figur 1 zeigt eine schematische Querschnittansicht einer erfindungsgemäßen Sprühvorrichtung. Der Kern der Sprühvorrichtung wird durch ein Ausströmrohr 2 mit einer Druckgaszuleitung 4 gebildet. In der Druckgaszuleitung 4 ist ein Drehventil 6 mit einer Durchführung 8 angeordnet. Die Durchführung 8 hat den gleichen Leitungsquerschnitt wie die Druckgaszuleitung 4 und verbindet die Druckgaszuleitung 4 in der geöffneten Stellung des Drehventils 6 und bildet eine Fortsetzung der Druckgaszuleitung 4. Das Drehventil 6 kann als konisches Bauteil in einen Ausströmkörper 10 des Ausströmrohrs 2 eingesetzt oder eingepresst sein. Das Drehventil 6 kann über einen Abzug 12 bedient werden.

Am Abzug 12 ist ein Rückstellelement 14 in Form einer elastischen Stahlfeder 14 vorgesehen. Im Inneren der Feder 14 befindet sich eine Federführungsstange. An einem Stutzen 16, der eine Fortführung des Ausströmrohrs 2 beziehungsweise der Druckgaszuleitung 4 bildet, ist ein Schlauch 18 befestigt, der an eine Druckgasquelle angeschlossen oder anschließbar ist. Der Ausströmkörper 10 und der restliche hintere Teil der Sprühvorrichtung sind in einem Gehäuse 20 untergebracht, das hinten einen Griff 22 nach Art eines Pistolengriffs bildet. Die Feder 14 ist mit dem Abzug 12 und dem Gehäuse 20 verbunden und sorgt dafür, dass der Abzug 12 in der gezeigten Stellung verbleibt und damit das Drehventil 6 geschlossen ist. Wenn das Lavage-System in eine Hand genommen wird, kann der Abzug 12 nach Art einer Pistole mit den Fingern der gleichen Hand gegen die Federkraft der Feder 14 bedient werden und so das Drehventil 6 geöffnet werden. Sobald der Abzug 12 losgelassen wird, schließt sich auch wieder das Ventilelement 6.

Das Ausströmrohr 2 mündet über eine Düse (nicht gezeigt) in ein Austragsrohr 24, das das Ausströmrohr 2 beabstandet umgibt. Wenn ein komprimiertes Gas bei geöffneten Drehventil 6 durch die Druckgaszuleitung 4 in das Austragsrohr 24 strömt, trifft es dort kurz hinter dem Ende des Ausströmrohrs 2 auf eine Verengung 26, die das Gas nach Art einer Düse beschleunigt. Am Ende des Austragsrohrs 24 ist ein Trichter 28 angeordnet.

Durch die Beschleunigung des Gases in Bereich der Verengung 26 wird im Bereich einer Einmündung 30 ein Unterdruck erzeugt. Durch diesen Unterdruck wird eine Flüssigkeit durch eine Flüssigkeitszuleitung 32 angesaugt und im Bereich des Einlasses von dem Gasstrom aus dem Ausströmrohr 2 mitgerissen. Dabei entstehen Tröpfchen der Flüssigkeit, die sich mit dem Gasstrom vermischen. Schließlich wird der mit den Flüssigkeitströpfchen vermischte Gasstrom durch den Trichter 28 ausgestoßen.

Am Griff 22 ist ein Drehschalter (nicht gezeigt) vorgesehen, mit dem ein Druckminderer 34 einstellbar ist, der in der Gaszuleitung 18 beziehungsweise dem Schlauch 18 angeordnet ist. Mit dem Druckminderer 34 kann der Gasdruck, der am Stutzen 16 ankommt und der durch die Druckgaszuleitung 4 auch in dem Bereich Wirkung entfaltet, in dem der Gasstrom mit der Flüssigkeit vermischt wird, eingestellt werden. Der Gasdruck ist auch für die Stärke beziehungsweise den Volumenstrom des Sprühkegels maßgeblich, der den Trichter 28 verlässt, beziehungsweise den die Sprühvorrichtung erzeugt. So ist es möglich die Stärke des Sprühkegels einzustellen. Wenn nur eine Stärke des Sprühkegels erwünscht ist und/oder eine besonders kostengünstige Variante der Sprühvorrichtung erwünscht ist, kann auf den Druckminderer 34 verzichtet werden.

Die medizinische Sprühvorrichtung kann als variables Lavage-System verwendet werden, bei dem die Stärke und damit die Wirkung des Sprühkegels einstellbar ist. Als Quelle für das Druckgas kann eine zentrale Durckgasanlage zum Einsatz kommen, ebenso kann der Schlauch 18 aber auch an einer CO₂-Patrone angeschlossen sein oder ein Kompressor zum Erzeugen des notwendigen Gasdrucks verwendet werden.

Die gesamte Sprühvorrichtung kann bis auf die Feder 14 und den Druckminderer 34 kostengünstig aus Kunststoff gefertigt werden.

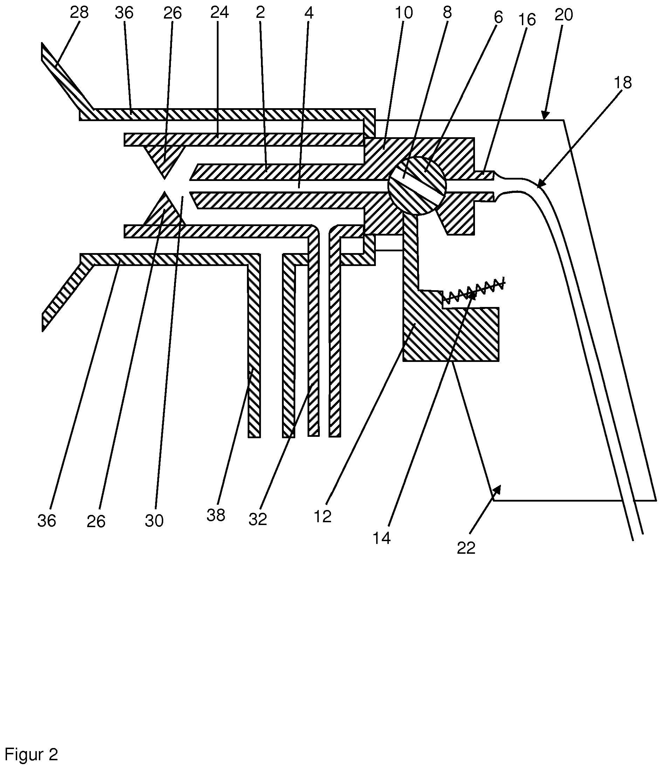

Figur 2 zeigt eine schematische Querschnittansicht einer alternativen erfindungsgemäßen Ausführungsform einer Sprühvorrichtung. Die Sprühvorrichtung ist grundsätzlich wie die nach Figur 1 aufgebaut, wobei der Druckminderer 34 weggelassen wurde und zusätzlich eine Absaugeinrichtung vorgesehen ist. Um das Austragsrohr 24 herum ist dazu ein Absaugrohr 36 angeordnet, das eine Absaugleitung 38 und eine Durchführung für die Flüssigkeitszuleitung 32 aufweist. Der Trichter 28 ist am äußersten Rohr 36, also hier dem Absaugrohr 36 angeordnet.

An der Absaugleitung 38 wird ein Unterdruck angelegt, beziehungsweise eine Pumpe angeschlossen, die einen geringen Unterdruck erzeugt, der dazu ausreicht, von der Vorderkante des Austragsrohrs 24 abtropfende Flüssigkeitsreste und die verbrauchte Flüssigkeit abzusaugen. Die Absaugeinrichtung wird bevorzugt dazu verwendet, die mit Gewebe kontaminierte Flüssigkeit abzusaugen. Hierfür reicht ein Unterdruck im Bereich von 50 mbar bis 400 mbar aus. Der schnelle Gas-Flüssigkeits-Strahl beziehungsweise - Kegel wird dabei nicht eingesaugt, da der Impuls des Strahls beziehungsweise des Kegels dafür zu hoch ist.

Die Ausführung der Sprühvorrichtungen nach den Figuren 1 und 2 durch ineinander angeordnete und beabstandete Rohre 2, 24, 36 dient einer besonders einfachen und kostengünstigen Fertigung der Sprühvorrichtungen. Die Rohre 2, 24, 36 mit den Zu- und Ableitungen 32, 38, dem Drehventil 6, dem Abzug 22, dem Gehäuse 20, dem Ausströmkörper 10, dem Stutzen 16 und dem Trichter 28 können als Kunststoffteile durch ein Spritzgussverfahren hergestellt werden und sind leicht zusammensetzbar.

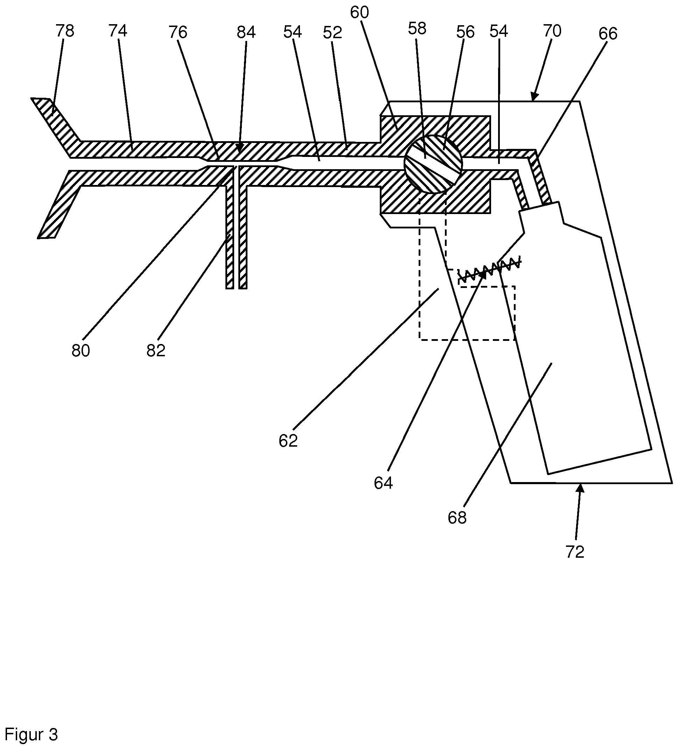

Figur 3 zeigt eine weitere nicht erfindungsgemäße Ausführungsform in schematischer Querschnittansicht. Die gezeigte medizinische Sprühvorrichtung umfasst ein Ausströmrohr 52, das einen vorderen Bereich einer Druckgaszuleitung 54 begrenzt. In der Druckgaszuleitung 54 ist ein Drehventil 56 angeordnet, das eine Durchführung 58 umfasst, die den gleichen Leitungsquerschnitt wie die Druckgaszuleitung 54 aufweist und die die Druckgaszuleitung 54 in der geöffneten Stellung des Drehventils 56 fortsetzt und verbindet. Das Drehventil 56 ist in gleicher Art und Weise wie das Drehventil 6 nach den Figuren 1 oder 2 in einen Ausströmkörper 60 eingesetzt und kann in gleicher Art und Weise über einen Abzug 62 mit einer Rückstellfeder 64 bedient werden.

Der Abzug 62 ist in Figur 3 durch gestrichelte Linien angedeutet, da er nicht in der Bildebene liegt. Es ist bevorzugt, wenn der Abzug 62 nicht in der gleichen ebene liegt wie die Durchführung 58 durch das Drehventil 56, um die baulichen Freiräume zur Gestaltung des Abzugs 62 und des Ausströmkörpers 60 zu gewährleisten. Der Ausströmkörper 60 benötigt dann nämlich keine Ausnehmung an der Stelle, an der die Beweglichkeit des Abzugs 62 dies erfordert. Eine solche Ausnehmung, wie sie in den Figuren 1 und 2 gezeigt ist, könnte den Druckgaszuleitungen 4, 54 in die Quere kommen. Zudem besteht die Gefahr, dass die Durchführung 8, 58 über diese Ausnehmung ungewollt mit der Umgebung verbunden werden könnte. Daher ist auch für die Ausführungsformen nach den Figuren 1 und 2, wie auch der nach Figur 3 bevorzugt vorgesehen, wenn der Abzug 12, 62 gegen die Ebene der Durchführung 8, 58 versetzt ist.

Über einen Anschluss 66, der eine Fortsetzung der Druckgaszuleitung 54 bildet, ist eine Druckgaspatrone 68 angeschlossen, die die Sprühvorrichtung mit einem Gasdruck versorgt. In dem Anschluss 66 ist ein von außen bedienbarer Druckminderer (nicht gezeigt) vorgesehen, mit dem der aus der Druckgaspatrone 68 in die Druckgaszuleitung 54 eingeleitete Gasdruck einstellbar ist. Die Druckgaspatrone 68 ist in dem Griffstück 72 eines Gehäuses 70 angeordnet.

Das Ausströmrohr 52 mündet über eine Verengung 76 in ein Austragsrohr 74. Das Austragsrohr 74 ist über einen Trichter 78 zur Ausrichtung und Begrenzung eines mit der Sprühvorrichtung zu erzeugenden Sprühkegels nach außen an die Umgebung geöffnet. Im Bereich der Verengung 76 ist eine Einmündung 80 vorgesehen, die eine Flüssigkeitszuleitung 82 mit der Durchführung in der Verengung 76 verbindet, wobei die Durchführung vorliegend als Teil des Austragsrohrs 74 aufgefasst wird, so dass die Verengung 76 vorliegend als Teil des Austragsrohrs 74 gilt. Die Flüssigkeitszuleitung 82 trifft senkrecht auf die Durchführung in der Verengung 76 und bildet dort eine Venturi-Düse 84.

Der Leitungsquerschnitt ist in der Verengung 76 im Vergleich zu den Leitungsquerschnitten der Druckgaszuleitung 54 und dem Austragsrohr 74 reduziert. Wenn das Gas aus der Druckgaspatrone 68 durch die Leitungen strömt, wird es im Bereich der Verengung 76 beschleunigt. Dadurch entsteht nach Bernoulli und Venturi ein Unterdruck in der Flüssigkeitszuleitung 82, durch den Flüssigkeit im Bereich der Einmündung 80 von der Gasströmung durch die Venturi-Düse 84 mitgerissen wird. Dadurch entsteht ein Gas-Flüssigkeits-Gemisch, das durch den Trichter 78 beziehungsweise die Öffnung des Austragsrohrs 74 ausgesprüht wird.

Das Ausströmrohr 52, das Austragsrohr 74, der Ausströmkörper 60, der Trichter 78, und der Anschluss 66 können einteilig aus Kunststoff gefertigt werden. Ebenso kann das Ventilelement 56 und der Abzug 62 einteilig aus Kunststoff gefertigt werden.

Für alle Kunststoffteile aller Ausführungsformen nach den Figuren 1, 2 und 3 kann bevorzugt ein thermoplastischer Kunststoff verwendet werden.

Die in der voranstehenden Beschreibung, sowie den Ansprüchen, Figuren und Ausführungsbeispielen offenbarten Merkmale der Erfindung können für die Verwirklichung der Erfindung in ihren verschiedenen Ausführungsformen wesentlich sein.

### Bezugszeichenliste

- 2, 52: Ausströmrohr
- 4, 54: Druckgaszuleitung
- 6, 56: Drehventil / Ventilelement
- 8, 58: Durchführung
- 10, 60: Ausströmkörper
- 12, 62: Abzug
- 14, 64: Feder
- 16: Stutzen
- 18: Schlauch
- 20, 70: Gehäuse
- 22, 72: Griff / Griffstück
- 24, 74: Austragsrohr
- 26, 76: Verengung / erste Düse
- 28, 78: Trichter
- 30, 80: Einmündung
- 32, 82: Flüssigkeitszuleitung
- 34: Druckminderer
- 36: Absaugrohr
- 38: Absaugleitung
- 66: Anschluss / Anschlussrohr
- 68: Druckgaspatrone
- 84: Venturi-Düse

## Patentansprüche

1. Medizinische Sprühvorrichtung, insbesondere ein Lavage-System, umfassend eine Druckgaszuleitung (4) zum Führen eines Gasstroms, eine Flüssigkeitszuleitung (32) zum Zuführen einer Flüssigkeit, ein Austragsrohr (24) mit einem offenen Rohrende zum Aussprühen von feinen Tröpfchen oder eines Sprühnebels eines Flüssigkeits-Gas-Gemischs und ein bedienbares Ventilelement (6), das in der Druckgaszuleitung (4) angeordnet ist, wobei in dem Austragsrohr (24) eine Verengung (26) der Querschnittsfläche vorgesehen ist, wobei eine Einmündung (30) im Bereich der Verengung (26) angeordnet ist, durch die die Flüssigkeit in den Gasstrom aus der Druckgaszuleitung (4) mündet, wobei die Verengung (26) in dem Austragsrohr (24) eine Düse bildet, **dadurch gekennzeichnet, dass** an einem Ende der Druckgaszuleitung (4), durch die das strömende Gas die Druckgaszuleitung (4) in Richtung der Verengung (26) verlässt, eine zweite Düse angeordnet ist.

2. Medizinische Sprühvorrichtung nach Anspruch 1, **dadurch gekennzeichnet, dass** im Bereich des offenen Rohrendes des Austragsrohrs (24) eine Absaugeinrichtung vorgesehen ist, die an eine Absaugleitung (38) angeschlossen ist, wobei bevorzugt die Absaugeinrichtung ein Absaugrohr (36) umfasst, in das die Absaugleitung (38) mündet, und besonders bevorzugt das Absaugrohr (36) das Austragsrohr (24) umgibt.

3. Medizinische Sprühvorrichtung nach Anspruch 1 oder 2, **dadurch gekennzeichnet, dass** an dem äußersten Rohrende, insbesondere an dem Austragsrohr (24) oder an einer Absaugeinrichtung beziehungsweise der Absaugeinrichtung ein Trichter (28) angeordnet ist, der zur Begrenzung und/oder Ausrichtung des erzeugten Sprühkegels geeignet ist.

4. Medizinische Sprühvorrichtung nach einem der vorangehenden Ansprüche, **dadurch gekennzeichnet, dass**
das Ventilelement (6) ein Drehventil (6) ist, insbesondere ein rotationssymmetrisches Drehventil (6), vorzugsweise ein konisches oder zylindrisches Drehventil (6) mit einem Durchgang (8), wobei das Drehventil (6) um die Drehachse drehbar in einer Ausnehmung in einem Ausströmkörper (10), der einen Teil der Druckgaszuleitung (4) definiert, gelagert ist, wobei bevorzugt das Drehventil (6) durch einen Abzug (12) bedienbar ist, besonders bevorzugt das Drehventil (6) einteilig mit dem Abzug (12) ausgebildet ist.

5. Medizinische Sprühvorrichtung nach einem der vorangehenden Ansprüche, **dadurch gekennzeichnet, dass**
das Ventilelement (6) durch ein Federelement (14) im geschlossenen Zustand gehalten wird, wobei vorzugsweise das Federelement (14) auf einer Seite an einem Abzug (12) beziehungsweise dem Abzug (12) befestigt ist.

6. Medizinische Sprühvorrichtung nach einem der vorangehenden Ansprüche, **dadurch gekennzeichnet, dass**
die Sprühvorrichtung im Wesentlichen aus Kunststoff besteht, bevorzugt thermoplastischem Kunststoff, besonders bevorzugt gammasterilisierbarem thermoplastischem Kunststoff, wobei insbesondere das Austragsrohr (24), das Ventilelement (6), die Druckgaszuleitung (4), ein Trichter (28) beziehungsweise der Trichter (28), ein Absaugrohr (36) beziehungsweise das Absaugrohr (36), ein Ausströmkörper (10) beziehungsweise der Ausströmkörper (10) und/oder die Verengung (26) aus Kunststoff bestehen.

7. Medizinische Sprühvorrichtung nach einem der vorangehenden Ansprüche, **dadurch gekennzeichnet, dass**
die Druckgaszuleitung (4) zumindest bereichsweise als Ausströmrohr (2) ausgebildet ist, das zumindest bereichsweise, insbesondere vollständig von dem Austragsrohr (24) umgeben ist.

8. Medizinische Sprühvorrichtung nach einem der vorangehenden Ansprüche, **dadurch gekennzeichnet, dass**
die Einmündung (30) bezogen auf die Strömungsrichtung vor der Düse (26) angeordnet ist.

9. Medizinische Sprühvorrichtung nach einem der vorangehenden Ansprüche, **dadurch gekennzeichnet, dass**
die Verengung (26) durch einen runden Ring mit dreieckiger Querschnittsfläche realisiert wird, wobei eine der Ecken der dreieckigen Querschnittsfläche in Richtung der Ringmitte orientiert ist.

10. Medizinische Sprühvorrichtung nach einem der Ansprüche 1 bis 7, **dadurch gekennzeichnet, dass**
die Einmündung in der Verengung ausgebildet ist, wobei bevorzugt die Düse eine Venturi-Düse ist.

11. Medizinische Sprühvorrichtung nach einem der vorangehenden Ansprüche, **dadurch gekennzeichnet, dass**
die Sprühvorrichtung einen Druckgasbehälter umfasst, der vorzugsweise mit flüssigem Kohlendioxid gefüllt ist, wobei der Druckgasbehälter bevorzugt in einem Griffstück (22) angeordnet ist.

12. Medizinische Sprühvorrichtung nach einem der vorangehenden Ansprüche, **dadurch gekennzeichnet, dass**
die Sprühvorrichtung einen einstellbaren Druckminderer (34) aufweist, mit dem der Gasdruck in die Druckgaszuleitung (4) einstellbar ist.

13. Medizinische Sprühvorrichtung nach einem der vorangehenden Ansprüche, **dadurch gekennzeichnet, dass**
die zweite Düse durch das Ausströmrohr (2) ausgeformt ist.

14. Sprühvorrichtungs-Kit umfassend eine medizinische Sprühvorrichtung nach einem der vorangehenden Ansprüche,
eine Gaszuleitung (18), insbesondere flexible Gaszuleitung (18), einen Druckregler (34) und einen Druckgasbehälter.

## Claims

1. Medical spray device, in particular a lavage system, comprising a compressed gas supply conduit (4) for generating a gas flow, a liquid supply conduit (32) for supplying a liquid, a dispensing tube (24) having an open tube end for spraying fine droplets or a spray mist of a liquid-gas mixture, and an operable valve element (6) that is arranged in the compressed gas supply conduit (4), wherein a constriction (26) of the cross-sectional area is provided in the dispensing tube (24) and a merging site (30) is arranged in the region of the constriction (26) through which the liquid merges into the gas flow from the compressed gas supply conduit (4), whereby the constriction (26) forms a nozzle in the dispensing tube (24), **characterised in that**
a second nozzle is arranged at the end of the compressed gas supply conduit (4) through which the flowing gas exits from the compressed gas supply conduit (4) in the direction of the constriction (26)

2. Medical spray device according to claim 1, **characterised in that**
an aspiration facility connected to an aspiration conduit (38) is provided in the region of the open tube end of the dispensing tube (24), whereby the aspiration facility preferably comprises an aspiration tube (36) into which the aspiration conduit (38) merges, and whereby the aspiration tube (36) particularly preferably surrounds the dispensing tube (24).

3. Medical spray device according to claim 1 or 2, **characterised in that**
a funnel (28) is arranged at the extreme tube end, in particular at the dispensing tube (24) or at an aspiration facility or at the aspiration facility, which funnel is suitable for limiting and/or aligning the spray cone produced.

4. Medical spray device according to any one of the preceding claims,
**characterised in that**
the valve element (6) is a rotary valve (6), in particular a rotationally symmetrical rotary valve (6), preferably a conical or cylindrical rotary valve (6), having a passage (8), whereby the rotary valve (6) is supported like in a bearing, such that it can be rotated, in a recess in an outflow body (10) that defines a part of the compressed gas supply conduit (4), whereby the rotary valve (6) preferably can be operated through a trigger (12), whereby it is particularly preferable for the rotary valve (6) and the trigger (12) to be provided as a same part.

5. Medical spray device according to any one of the preceding claims,
**characterised in that**
the valve element (6) is maintained in the closed state by means of a spring element (14), whereby the spring element (14) preferably is attached on one side on a trigger (12) or on the trigger (12).

6. Medical spray device according to any one of the preceding claims,
**characterised in that**
the spray device consists essentially of plastic material, preferably of thermoplastic material, particularly preferably of thermoplastic material suited for gamma-sterilisation, whereby in particular the dispensing tube (24), the valve element (6), the compressed gas supply conduit (4), a/the funnel (28), an/the aspiration tube (36), an/the outflow body (10) and/or the constriction (26) consist of plastic material.

7. Medical spray device according to any one of the preceding claims,
**characterised in that**
the compressed gas supply conduit (4) is provided, at least over regions thereof, as outflow tube (2) that is surrounded, at least over regions thereof, in particular fully, by the dispensing tube (24).

8. Medical spray device according to any one of the preceding claims,
**characterised in that**
the merging site (30) is arranged upstream of the nozzle (26) with respect to the direction of flow.

9. Medical spray device according to any one of the preceding claims,
**characterised in that**
the constriction (26) is implemented through a circular ring having a triangular cross-sectional area, whereby one of the corners of the triangular cross-sectional area is oriented towards the middle of the ring.

10. Medical spray device according to any one of the claims 1 to 7, **characterised in that**
the merging site is situated in the constriction, whereby the nozzle preferably is a Venturi nozzle.

11. Medical spray device according to any one of the preceding claims,
**characterised in that**
the spray device comprise a compressed gas container that is preferably filled with liquid carbon dioxide, whereby the compressed gas container is preferably arranged in a handle part (22).

12. Medical spray device according to any one of the preceding claims,
**characterised in that**
the spray device comprises a settable pressure reducer (34) by which the gas pressure in the compressed gas supply conduit (4) is adjustable.

13. Medical spray device according to any one of the preceding claims,
**characterised in that**
the second nozzle is formed through the outflow tube (2).

14. Spray device kit comprising a medical spray device according to any one of the preceding claims, a gas supply conduit (18), in particular flexible gas supply conduit (18), a pressure regulator (34), and a compressed gas container.

## Revendications

1. Dispositif de pulvérisation médical, en particulier, système de lavage, comprenant une conduite d'alimentation de gaz sous pression (4) pour emmener un flux de gaz, une conduite d'alimentation de liquide (32) pour alimenter un liquide, un tuyau de distribution (24) avec une extrémité de tuyau ouverte pour la pulvérisation de fines gouttelettes ou d'un brouillard de pulvérisation d'un mélange liquide-gaz et un élément de soupape (6) actionnable qui est disposé dans la conduite d'alimentation de gaz sous pression (4), où un rétrécissement (26) de la surface de la section transversale est prévu dans le tuyau de distribution (24),
où une arrivée (30) est aménagée dans la région du rétrécissement (26) par laquelle le liquide débouche dans le flux de gaz hors de la conduite d'alimentation de gaz sous pression (4), où le rétrécissement (26) forme une buse dans le tuyau de distribution (24), **caractérisé en ce**
**qu'**une deuxième buse est disposée à une extrémité de la conduite d'alimentation de gaz sous pression (4) par laquelle le gaz s'écoulant quitte la conduite d'alimentation de gaz sous pression (4) en direction du rétrécissement (26).

2. Dispositif de pulvérisation médical selon la revendication 1, **caractérisé en ce que**
dans la région de l'extrémité de tuyau ouverte du tuyau de distribution (24), il est prévu un dispositif d'aspiration qui est raccordé à une conduite d'aspiration (38), où, de préférence, le dispositif d'aspiration comprend un tuyau d'aspiration (36) dans lequel débouche la conduite d'aspiration (38) et de manière particulièrement préférée, le tuyau d'aspiration (36) entoure le tuyau de distribution (24).

3. Dispositif de pulvérisation médical selon la revendication 1 ou la revendication 2, **caractérisé en ce**
**qu'**un entonnoir (28) est disposé à l'extrémité le plus à l'extérieur du tuyau, en particulier, sur le tuyau de distribution (24) ou au niveau d'un dispositif d'aspiration, respectivement, du dispositif d'aspiration, qui est approprié pour la délimitation et/ou l'orientation du cône de pulvérisation créé.

4. Dispositif de pulvérisation médical selon l'une des revendications précédentes, **caractérisé en ce que**
l'élément de soupape (6) est une soupape rotative (6), en particulier, une soupape rotative (6) symétrique en rotation, de préférence, une soupape rotative (6) conique ou cylindrique avec un passage (8), où la soupape rotative (6) est logée, en pouvant être mise en rotation autour de l'axe de rotation, dans une cavité dans un corps de déversement (10) qui définit une partie de la conduite d'alimentation de gaz sous pression (4), où, de préférence, la soupape de rotation (6) peut être actionnée par une gâchette (12), de manière particulièrement préférée, la soupape de rotation (6) est conçue d'un seul tenant avec la gâchette (12).

5. Dispositif de pulvérisation médical selon l'une des revendications précédentes, **caractérisé en ce que**
l'élément de soupape (6) est maintenu dans l'état fermé par un élément de ressort (14), où, de préférence, l'élément de ressort (14) est fixé sur un côté au niveau d'une gâchette (12), respectivement, de la gâchette (12).

6. Dispositif de pulvérisation médical selon l'une des revendications précédentes, **caractérisé en ce que**
le dispositif de pulvérisation est principalement constitué de matière plastique, de préférence, une matière plastique thermoplastique, de manière particulièrement préférée, de matière plastique thermoplastique pouvant être stérilisée par un rayonnement gamma, où, en particulier, le tuyau de distribution (24), l'élément de soupape (6), la conduite d'alimentation de gaz sous pression (4), un entonnoir (28), respectivement, l'entonnoir (28), un tuyau d'aspiration (36), respectivement le tuyau d'aspiration (36), un corps d'écoulement de sortie (10), respectivement le corps d'écoulement de sortie (10) et/ou le rétrécissement (26) sont constitués de matière plastique.

7. Dispositif de pulvérisation médical selon l'une des revendications précédentes, **caractérisé en ce que**
la conduite d'alimentation de gaz sous pression (4) est conçue au moins par endroits sous forme d'un tuyau d'écoulement (2) qui est entouré au moins par endroits, notamment, totalement par le tuyau de distribution (24).

8. Dispositif de pulvérisation médical selon l'une des revendications précédentes, **caractérisé en ce**
**qu'**une arrivée (30) est disposée avant la buse (26) par rapport au sens d'écoulement.

9. Dispositif de pulvérisation médical selon l'une des revendications précédentes, **caractérisé en ce que**
le rétrécissement (26) est réalisé par un anneau circulaire avec une surface de section transversale triangulaire, où un des angles de la surface de section transversale triangulaire est orienté en direction du milieu de l'anneau.

10. Dispositif de pulvérisation médical selon l'une des revendications 1 à 7, **caractérisé en ce que**
l'arrivée est conçue dans le rétrécissement, où, de préférence, la buse est une buse venturi.

11. Dispositif de pulvérisation médical selon l'une des revendications précédentes, **caractérisé en ce que**
le dispositif de pulvérisation comprend un récipient de gaz sous pression qui est de préférence rempli de dioxyde de carbone liquide, où le récipient de gaz sous pression est disposé de préférence dans une poignée (22).

12. Dispositif de pulvérisation médical selon l'une des revendications précédentes, **caractérisé en ce que**
le dispositif de pulvérisation présente un réducteur de pression (34) réglable avec lequel la pression du gaz peut être réglée dans la conduite de gaz sous pression (4).

13. Dispositif de pulvérisation médical selon l'une des revendications précédentes, **caractérisé en ce que**
la deuxième buse est façonnée par le tuyau d'écoulement de sortie (2).

14. Ensemble de dispositif de pulvérisation comprenant un dispositif de pulvérisation médical selon l'une des revendications précédentes,
une conduite d'alimentation de gaz (18), notamment une conduite d'alimentation de gaz (18) souple, un régulateur de pression (34) et un récipient de gaz sous pression.
